# EUROPÄISCHE PATENTSCHRIFT

(11) **EP 0 416 069 B1**
(45) Veröffentlichungstag und Bekanntmachung des Hinweises auf die Patenterteilung: **28.07.1993**
(21) Anmeldenummer: 90904600.5
(22) Anmeldetag: 16.03.1990
(51) Int. Cl.: C07C 69/736, C07C 67/343, A61K 31/215, C07C 59/68, C07C 43/23, C07C 59/70, C07D 207/16

(54) **NEUE LEUKOTRIEN-B 4 -DERIVATE, VERFAHREN ZU IHRER HERSTELLUNG UND IHRE VERWENDUNG ALS ARZNEIMITTEL**
NEW LEUCOTRIENE B 4 DERIVATIVES, A METHOD OF PREPARING THEM, AND THEIR USE AS DRUGS
NOUVEAUX DERIVES DE LEUCOTRIENE B 4 , PROCEDE POUR LEUR PREPARATION, ET LEUR EMPLOI COMME MEDICAMENTS

(30) Priorität: 17.03.1989 DE 3909326
(43) Veröffentlichungstag der Anmeldung: 13.03.1991
(73) Patentinhaber: SCHERING AKTIENGESELLSCHAFT, 13342 Berlin (DE)
(72) Erfinder: HEINDL, Josef, D-1000 Berlin 38 (DE); SKUBALLA, Werner, D-1000 Berlin 28 (DE); BUCHMANN, Bernd, D-1000 Berlin 21 (DE); FRÖHLICH, Wolfgang, D-1000 Berlin 31 (DE); EKERDT, Roland, D-1000 Berlin 28 (DE)
(86) Internationale Anmeldenummer: DE9000209
(87) Internationale Veröffentlichungsnummer: WO9011272

(56) Entgegenhaltungen:
- EP-A- 0 109 225
- EP-A- 0 296 580

## Beschreibung

Die Erfindung betrifft neue Leukotrien-B₄-Derivate, Verfahren zu ihrer Herstellung sowie ihre Verwendung als Arzneimittel.

Leukotrien B₄ (LTB₄) wurde 1979 von B. Samuelsson und Mitarbeiter als Metabolit der Arachidonsäure entdeckt. Bei der Biosynthese wird durch das Enzym 5-Lipoxygenase zunächst als zentrales Zwischenprodukt das Leukotrien A₄ gebildet, das dann durch eine spezifische Hydrolase in das LTB₄ umgewandelt wird.

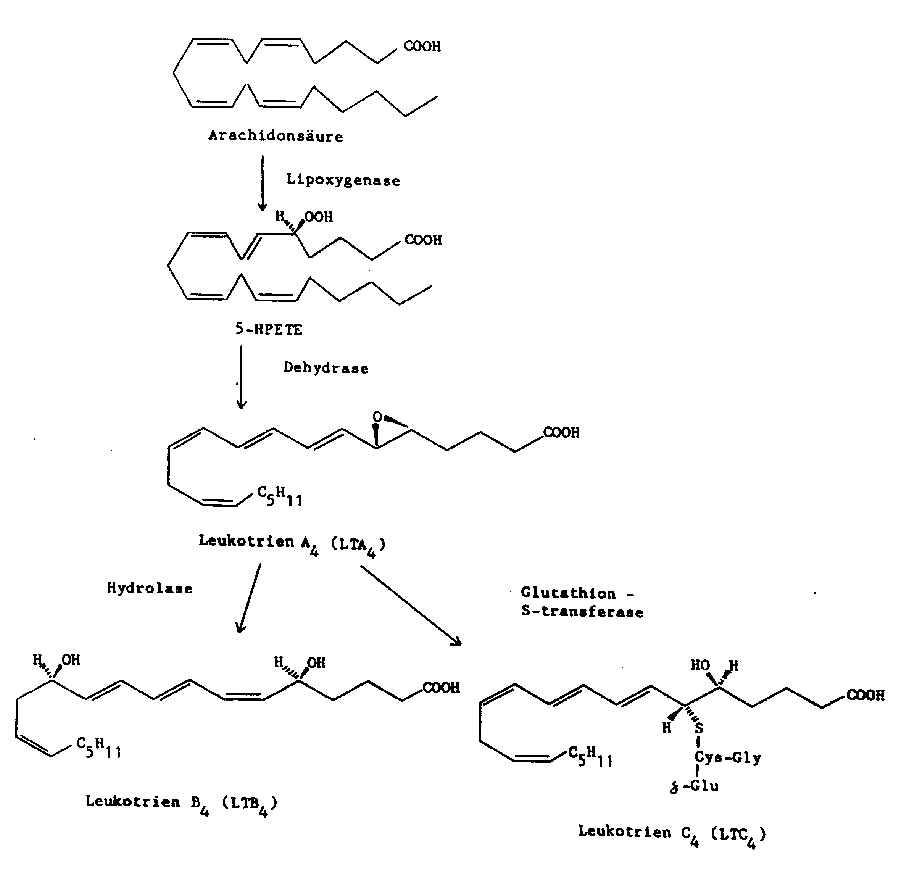

Die Nomenklatur der Leukotriene kann folgenden Arbeiten entnommen werden: a) B. Samuelsson et al., Prostaglandins 19, 645 (1980); 17, 785 (1979). b) C.N. Serhan et al., Prostaglandins 34, 201 (1987).

Die physiologische und insbesondere die pathophysiologische Bedeutung des Leukotrien B₄ wird in einigen neueren Arbeiten zusammengefaßt: a) The Leukotrienes, Chemistry and Biology eds. L.W. Chakrin, D.M. Bailey, Academic Press 1984. b) J.W. Gillard et al., Drugs of the Future 12, 453 (1987). c) B. Samuelsson et al., Science 237, 1171 (1987). d) C.W. Parker, Drug Development Research 10, 277 (1987). Hieraus ergibt sich, daß LTB₄ ein wichtiger Entzündungsmediator für entzündliche Erkrankungen ist, bei denen Leukozyten in das erkrankte Gewebe einwandern.

Vom LTB₄ weiß man, daß es die Adhäsion von Leukozyten an die Blutgefäßwand verursacht. LTB₄ ist chemotaktisch wirksam, d.h. es löst eine gerichtete Wanderung von Leukozyten in Richtung eines Gradienten steigender Konzentration aus. Außerdem verändert es aufgrund seiner chemotaktischen Aktivität indirekt die vasculäre Permeabilität, wobei ein Synergismus mit Prostaglandin E₂ beobachtet wurde. LTB₄ spielt offensichtlich bei entzündlichen, allergischen und immunologischen Prozessen eine entscheidende Rolle.

Leukotriene und insbesondere LTB₄ sind an Hauterkrankungen beteiligt, die mit entzündlichen Prozessen (erhöhte Gefäßpermeabilität und Ödembildung, Zellinfiltration), erhöhter Proliferation der Hautzellen und Juckreiz einhergehen, wie beispielsweise bei Ekzemen, Erythemen, Psoriasis, Pruritus und Akne. Pathologisch erhöhte Leukotrienkonzentration sind an der Entstehung vieler Dermatitiden entweder ursächlich beteiligt, oder es besteht ein Zusammenhang zwischen der Persistenz der Dermatitiden und den Leukotrienen. Deutlich erhöhte Leukotrienkonzentration wurden beispielsweise in der Haut von Patienten mit Psoriasis oder atopischer Dermatitis gemessen.

Weiterhin sind Leukotriene und LTB₄ insbesondere bei Arthritis chronischer Lungenerkrankung (z.B. Asthma), Rhinitis und entzündlichen Darmerkrankungen beteiligt.

Antagonisten gegen LTB₄ selbst oder Inhibitoren jener Enzyme, die an der Synthese des LTB₄ beteiligt sind, können als spezifische Heilmittel, besonders gegen Krankheiten, die mit Entzündungen und allergischen Reaktionen einhergehen, wirksam sein.

Neben therapeutischen Möglichkeiten, die sich aus einer Antagonisierung des LTB₄ mit LTB₄-Analoga ableiten lassen, konnte kürzlich auch die Nützlichkeit und potentielle Anwendung von Leukotrien B₄-Agonisten zur Behandlung von Pilzerkrankungen der Haut gezeigt werden (H. Kayama, Prostaglandins 34, 797 (1988)).

Der Ersatz der chemisch und metabolisch labilen cis-Δ⁶^{,7}-Doppelbindung des LTB₄ durch einen 1,2-substituierten Phenylring führt zu den stabileren 6,7-Interphenylen-Leukotrienen, wobei je nach Strukturveränderung der funktionellen Gruppen und je nach Gewebeart Antagonisten, Agonisten und Partialantagonisten erhalten werden. Es wurde nun gefunden, daß durch die Substitution der 5-Hydroxymethylgruppe durch ein Sauerstoffatom und durch weitere Derivatisierung der funktionellen Gruppen LTB₄-Analoga erhalten werden, die die Wirkung des natürlichen LTB₄ stark antagonisieren. Wirkdauer und Selektivität der neuen Verbindungen konnten durch geringere Oxidationsempfindlichkeit oder fehlende Laktonisierungstendenz aufgrund der nicht vorhandenen 5-Hydroxygruppe weiter verbessert werden.

Die Erfindung betrifft neue Leukotrien-B₄-Derivate der Formel I, 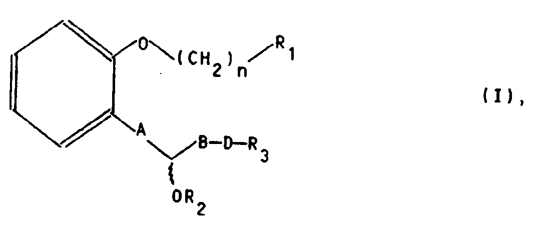
worin n = 1-10,
- R₁: den Rest CH₂OH, den Rest COOR₄ mit R₄ in der Bedeutung eines Wasserstoffatoms, eines Alkylrestes mit 1-10 C-Atomen, eines Cycloalkylrestes mit 3-10 C-Atomen, eines gegebenenfalls durch 1-2 Chlor, Brom, Phenyl, Alkyl mit 1-4 C-Atomen, Chlormethyl, Fluormethyl, Trifluormethyl, Carboxy, C₁-C₄-Alkoxy oder Hydroxy substituierten Arylrestes mit 6-10 C-Atomen, eines -CH₂-CO-Aryl-Restes mit 6-10 C-Atomen für Aryl oder eines 5-6-gliedrigen heterocyclischen Restes mit wenigstens 1 Heteroatom, den Rest CONHR₅ mit R₅ in der Bedeutung eines Alkanoyl- oder Alkansulfonylrestes mit 1-10 C-Atomen oder des Restes R₄ oder den Rest CONR₆R₇, wobei R₆ und R₇ Alkyl mit 1-10 C-Atomen oder gemeinsam einen Alkylenrest mit 3-6 C-Atomen bedeuten,

- A: eine cis,trans- oder trans,trans-CH=CH-CH=CH- oder Tetramethylengruppe,
- B: eine geradkettige oder verzweigtkettige gesättigte oder ungesättigte Alkylengruppe mit bis zu 10 C-Atomen, die gegebenenfalls durch Fluor substituiert sein kann, oder die Gruppe 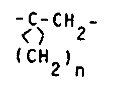 mit n = 1, 2 oder 3,
- D: eine Direktverbindung, Sauerstoff, Schwefel, eine -C≡C-Gruppe oder eine -CH=CR₈-Gruppe mit R₈ als Wasserstoff, C₁-C₅-Alkyl, Chlor oder Brom,
- B und D: gemeinsam eine direkte Bindung,

- R₂: ein Wasserstoffatom oder einen Säurerest einer organischen Säure mit 1-15 C-Atomen und
- R₃: ein Wasserstoffatom, einen Alkylrest mit 1-10 C-Atomen, einen durch Chlor oder Brom substituierten Alkylrest mit 1-10 C-Atomen, einen Cycloalkylrest mit 3-10 C-Atomen, einen gegebenenfalls durch 1-2 Chlor-, Brom, Phenyl, Alkyl mit 1-4 C-Atomen, Chlormethyl, Fluormethyl, Trifluormethyl, Carboxy, C₁-C₄-Alkoxy oder Hydroxy substituierten Arylrest mit 6-10 C-Atomen oder einen 5-6-gliedrigen heterocyclischen Rest mit wenigstens 1 Heteroatom bedeuten und, falls R₅ die Bedeutung eines Wasserstoffatom hat, deren Salze mit physiologisch verträglichen Basen und deren Cyclodextrinclathrate.

Als Alkylgruppen R₄, R₆ und R₇ kommen gerad- oder verzweigtkettige Alkylgruppen mit 1-10 C-Atomen in Betracht, wie beispielsweise Methyl, Ethyl, Propyl, Butyl, Isobutyl, tert.-Butyl, Pentyl, Neopentyl, Hexyl, Meptyl, Decyl. Die Alkylgruppen R₄ können gegebenenfalls ein- bis mehrfach substituiert sein durch Halogenatome, Alkoxygruppen, gegebenenfalls substituierte Aryl- bzw. Aroylgruppen, Dialkylamino und Trialkylammonium, wobei die einfache Substitution bevorzugt sein soll. Als Substituenten seien beispielsweise genannt Fluor, Chlor oder Brom, Phenyl, Dimethylamino, Diethylamino, Methoxy, Ethoxy. Als bevorzugte Alkylgruppen R₄ sind solche mit 1-4 C-Atomen zu nennen.

Wenn R₆ und R₇ gemeinsam mit dem N-Atom des Restes R₁ = CONR₆R₇ einen Alkylenrest mit 3-6 C-Atomen bedeutet, sind folgende Reste gemeint: Azetidin, Pyrrolidin, Piperidin, Azepin.

Als Arylgruppen R₄ kommen sowohl substituierte wie auch unsubstituierte Arylgruppen in Betracht, wie beispielsweise Phenyl, 1-Naphthyl und 2-Naphthyl, die jeweils substituiert sein können durch 1-3 Halogenatome (F, Cl, Br), eine Phenylgruppe, 1-3 Alkylgruppen mit jeweils 1-4 C-Atomen, eine Chlormethyl-, Fluormethyl-, Trifluormethyl,- Carboxyl-, Hydroxy- oder Alkoxygruppe mit 1-4 C-Atomen. Bevorzugte Substituenten in 3- und 4-Stellung am Phenylring sind zum Beispiel Fluor, Chlor, Alkoxy oder Trifluormethyl, in 4-Stellung dagegen Hydroxy. Der Arylrest im -CH₂-CO-Aryl-Rest stellt einen Phenyl- oder Naphtylrest dar, der gegebenenfalls durch Halogen (Chlor, Brom), Trifluormethyl oder Phenyl substituiert sein kann.

Die Cycloalkylgruppe R₄ kann im Ring 3-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl-, Cyclohexyl, Methylcyclohexyl.

Als heterocyclische Gruppen R₄ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, oxazolyl, Thiazolyl, Pyrimidinyl, Pyridazinyl, Pyrazinyl, 3-Furyl, 3-Thienyl, 2-Tetrazolyl u.a.

Als Säurerest R₅ kommen physiologisch verträgliche Säurereste in Frage. Bevorzugte Säuren sind organische Carbonsäuren und Sulfonsäuren mit 1-10 Kohlenstoffatomen, die der aliphatischen, cycloaliphatischen, aromatischen, aromatisch-aliphatischen und heterocyclischen Reihe angehören. Diese Säuren können gesättigte, ungesättigte und/oder mehrbasisch und/oder in üblicher Weise substituiert sein. Als Beispiele für die Substituenten seien Alkyl-, Hydroxy-, Alkoxy-, oxo- oder Aminogruppen oder Halogenatome erwähnt. Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Trimethylessigsäure, Diethylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyesigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy,- Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Acylreste und Alkansulfonylreste werden solche mit bis zu 6 Kohlenstoffatomen betrachtet. Als Sulfonsäuren kommen beispielsweise Methansulfonsäure, Ethansulfonsäure, Isopropansulfonsäure, β-Chlorethansulfonsäure, Butansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Diethylaminosulfonsäure, N,N-Bis-(β-chlorethyl)-aminosulfonsäure, N,N-Diisobutylaminosulfonsäure, N,N-Dibutylaminosulfonsäure, Pyrrolidino-, Piperidino-, Piperazino-, N-Methylpiperazino- und Morpholinosulfonsäure in Frage.

Als Alkylgruppen R₃ kommen gerad- und verzweigtkettige, gesättigte und ungesättigte Alkylreste, vorzugsweise gesättigte, mit 1-10, insbesondere 1-6 C-Atomen, in Frage, die gegebenenfalls durch gegebenenfalls substituiertes Aryl substituiert sein können. Beispielsweise genannt seien Methyl-, Ethyl-, Propyl-, Butyl-, Isobutyl-, tert.,-Butyl-, Pentyl-, Hexyl-, Heptyl-, octyl-, Butenyl-, Isobutenyl-, Propenyl-, Pentenyl-, Benzyl-, m- und p-Chlorbenzylgruppen. Sind die Alkylgruppen R₃ Halogen-substituiert, kommen als Halogene Fluor, Chlor und Brom in Frage.

Die Cycloalkylgruppe R₃ kann im Ring 3-10, vorzugsweise 3-6 Kohlenstoffatome enthalten. Die Ringe können durch Alkylgruppen mit 1-4 Kohlenstoffatomen substituiert sein. Beispielsweise genannt seinen Cyclopropyl, Cyclobutyl, Cyclopentyl, Cyclohexyl, Methyl-cyclohexyl.

Als substituierte bzw. unsubstituierte Arylgruppen R₃ kommen beispielsweise in Betracht: Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen eine Chlormethyl-, Fluormethyl-, Trifluormethyl-, Carboxyl-, C₁-C₄-Alkoxy- oder Hydroxygruppe. Bevorzugt ist die Substitution in 3- und 4-Stellung am Phenylring zum Beispiel durch Fluor, Chlor, Alkoxy oder Trifluormethyl oder in 4-Stellung durch Hydroxy.

Als heterocyclische Gruppen R₃ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, oxazolyl, Thiazolyl, Pyrimidinyl, Pyridazinyl, Pyrazinyl, 3-Furyl, 3-Thienyl, u.a.

Als Alkylengruppe B kommen geradkettige oder verzweigtkettige, gesättigte und ungesättigte Alkylenreste mit bis zu 10 C-Atomen, vorzugsweise gesättigte mit 1-10, insbesondere mit 1-5 C-Atomen, in Frage, die gegebenenfalls durch Fluoratome substituiert sein können. Beispielsweise seien genannt: Methylen, Fluormethylen, Difluormethylen, Ethylen, 1,2-Propylen, Ethylethylen, Trimethylen, Tetramethylen, Pentamethylen, 1.1-Difluorethylen, 1-Fluorethylen, 1-Methyltetramethylen, 1-Methyl-tri-methylen, 1-Methylen-ethylen, 1-Methylen-tetramethylen.

Als Säurereste R₂ kommen physiologisch verträgliche Säurereste in Frage. Bevorzugte Säuren sind organische Carbonsäuren und Sulfonsäuren mit 1-15 Kohlenstoffatomen, die der aliphatischen, cyclo-aliphatischen, aromatischen, aromatisch-aliphatischen oder heterocyclischen Reihe angehören. Diese Säuren können gesättigt, ungesättigt und/oder mehrbasisch und/oder in üblicher Weise substituiert sein. Als Beispiele für die Substituenten seien Alkyl-, Hydroxy-, Alkoxy, oxo- oder Aminogruppen oder Halogenatome erwähnt.

Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsaure, Diethylessigsäure, tert.-Butylessigsäure, Cyclopentylessigsäure, Cyclohexylessigsäure, Cyclohexancarbonsäure, Phenylessigsäure, Phenoxyesigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy,- Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Säurereste R₂ kommen Acylreste mit bis zu 10 Kohlenstoffatomen in Betracht.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natrium- und Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin usw.

Die Leukotrien-B₄-Derivate der Formel I bilden mit α-, β-, γ-Cyclodextrin die bereits genannten Cyclodextrinclathrate.

Die Erfindung beinhaltet außerdem ein Verfahren zur Herstellung der Leukotrien-B₄-Derivate der Formel I, das dadurch gekennzeichnet ist, daß man ein Vinylhalogenid der Formel II oder III, 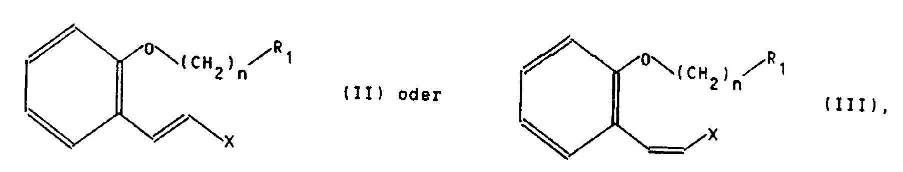
worin R₁ und n die bereits angegebenen Bedeutungen haben und X ein Jod- oder Bromatom ist, mit einer zinnorganischen Verbindung der Formel IV,

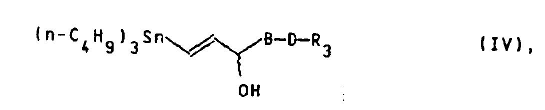

Worin B, D und R₃ die bereits angegebenen Bedeutungen haben, mit Hilfe eines Palladium-Katalysators umsetzt und gegebenenfalls anschließend in beliebiger Reihenfolge Enantiomere trennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe (R₁=COOR₄) verseift und/oder reduziert und/oder eine Carboxylgruppe (R₄=H) verestert und/oder eine freie Carboxylgruppe (R₄=H) in ein Amid überführt oder eine Carboxygruppe mit einer physiologisch verträglichen Base in ein Salz überführt.

Die Ausgangsverbindungen der Formel II und III lassen sich nach folgenden Methoden herstellen:

### Methode A [analog K. Takai et al. J. Amer. Chem. Soc. 108 (1986) 7408]:

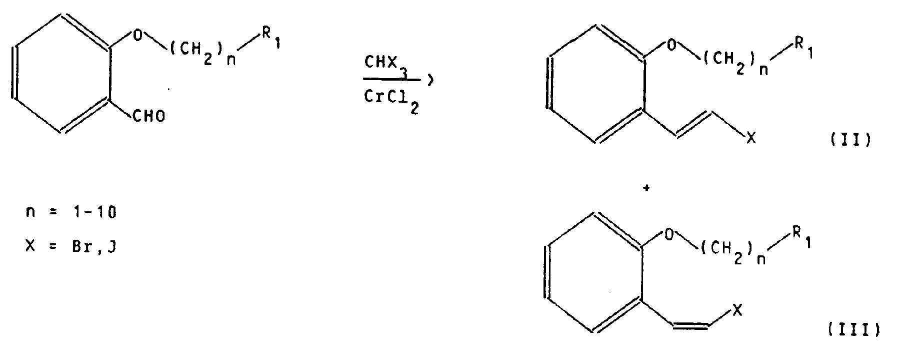

### Methode B [analog R.A. Haack et al. Tetrahedron Letters 29 (1988) 2783]:

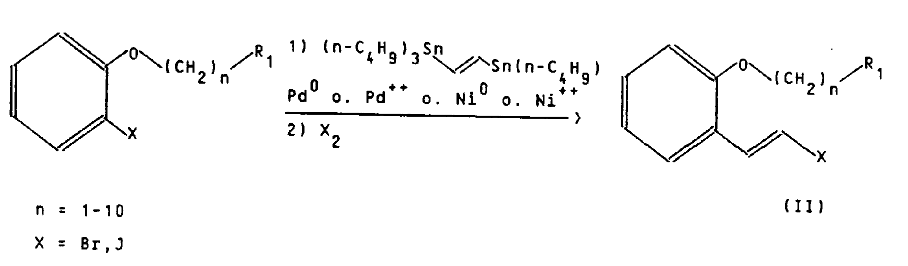

Für die Umsetzung nach Methode B und für die Umsetzung von Verbindungen der Formeln II und III mit IV geeignete Nickel- und Palladiumkatlysatoren sind beispielsweise 1,3-Bis-(diphenylphosphino)-propan-nickel-II-chlorid, Tetrakistriphenylphosphin-nickel, Bis-tri-o-tolylphosphin-palladium-II-chlorid, Bis-tri-phenylphosphin-palladium-II-chlorid, Tetrakis-tri-phenylphosphin-palladium, 1,1'-Bis-(diphenylphosphino)-ferrocen-palladium-II-chlorid und Bis-(acetonitril)-palladium-II-chlorid.

Die Ausgangsverbindung der Formel IV werden wie folgt hergestellt: 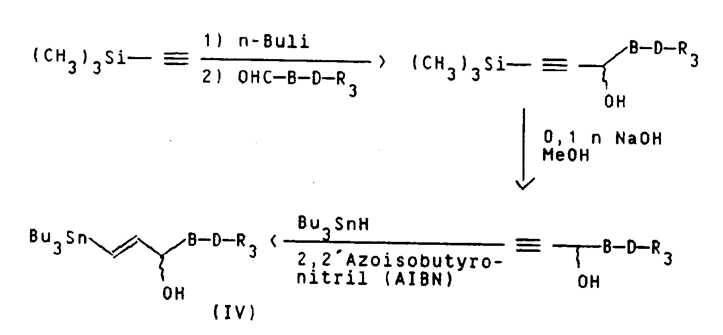
worin B, D und R₃ die oben angegebenen Bedeutungen haben.

Die Umsetzung von 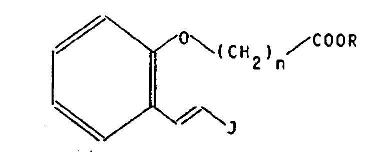
mit 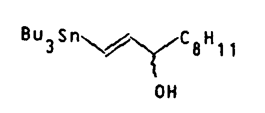
in Gegenwart von Pd zu 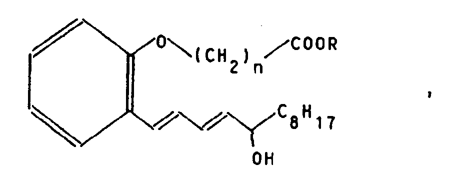
einer Verbindung nach Formel I,
wird entsprechend J.K. Stille et al. J. Amer. Chem. Soc. 109 (1987) 2143 durchgeführt.

Die Verbindung 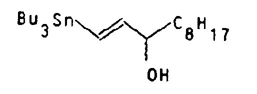
läßt sich mit Hilfe der Sharpless-Epoxidation {Y. Gao ....... and K.B. Sharpless J. Amer. Chem. Soc. 109 (1987) 5765} zu 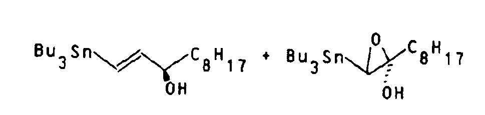
umsetzen.

Nach chromatographischer Trennung erhält man 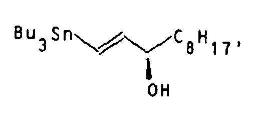
das mit 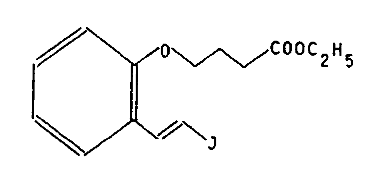
in Gegenwart von Pd das β-Isomere 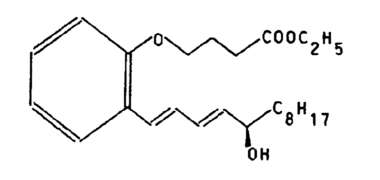
liefert.

Die Ausgangsverbindungen der Methode A 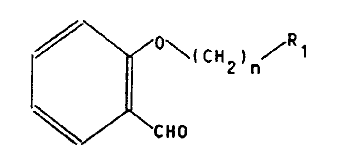
werden aus Salicylaldehyd durch Umsetzung mit Bromalkansäureestern in Gegenwart von NaH erhalten.

Die Ausgangsverbindungen der Methode B 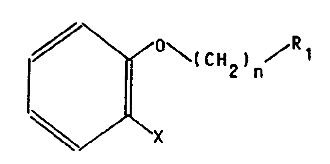
werden aus o-Jod- oder o-Bromphenol und w-Bromalkansäureestern (alkan=ethan, butan, pentan) in DMF in Gegenwart von Cs₂CO₃ bei Raumtemperatur in 80 %iger Ausbeute erhalten.

Alle hergestellten Verbindungen der Formel I bis auf die Verbindung aus Beispiel 3 stellen Racemate dar. Die Racemate können an einer "optisch aktiven" Säule (Chiralcel-OD-Säule) getrennt werden.

Die Reduktion zu den Verbindungen der Formel I mit R₁ in der Bedeutung einer -CH₂OH-Gruppe wird mit einem für die Reduktion von Estern oder Carbonsäuren geeigneten Reduktionsmittel wie beispielsweise Lithiumaluminiumhydrid, Diisobutylaluminiumhydrid usw. durchgeführt. Als Lösungsmittel kommen Diethylether, Tetrahydrofuran, Dimethoxyethan, Toluol usw. in Frage. Die Reduktion wird bei Temperaturen von -30 °C bis zur Siedetemperatur des verwendeteten Lösungsmittels, vorzugsweise 0 °C bis 30 °C vorgenommen.

Die Veresterung der Alkohole der Formel I (R₂ = H) erfolgt in an sich bekannter Weise. Zum Beispiel erfolgt die Veresterung dadurch, da8 man ein Säurederivat, vorzugsweise ein Säurehalogenid oder Säureanhydrid, in Gegenwart einer Base wie beispielsweise Na-Hydrid, Pyridin, Triethylamin, Tributylamin oder 4-Dimethylaminopyridin mit einem Alkohol der Formel I umsetzt. Die Umsetzung kann ohne Lösungsmittel oder in einem inerten Lösungsmittel, vorzugsweise Aceton, Acetonitril, Dimethylacetamid, DMSO bei Temperaturen über oder unter Raumtemperatur, zum Beispiel zwischen -80 °C bis 100 °C, vorzugsweise bei Raumtemperatur, vorgenommen werden.

Die Verseifung der Ester der Formel I wird nach den dem Fachmann bekannten Methoden durchgeführt, wie beispielsweise mit basischen Katalysatoren. Die Verbindungen der Formel I können durch die üblichen Trennmethoden in die optischen Isomeren getrennt werden.

Die Verseifung der Acylgruppen erfolgt beispielsweise mit Alkali- oder Erdalkali-carbonaten oder -hydroxyden in einem Alkohol oder in der wässrigen Lösung eines Alkohols. Als Alkohol kommen aliphatische Alkohole in Betracht, wie z.B. Methanol, Ethanol, Butanol usw., vorzugsweise Methanol. Als Alkalicarbonate und -hydroxyde seien Kalium- und Natriumsalze genannt. Bevorzugt sind die Kaliumsalze. Als Erdkalicarbonate und -hydroxide sind beispielsweise geeignet Calciumcarbonat, Calciumhydroxyd und Bariumcarbonat. Die Umsetzung erfolgt bei -10 °C bis +70 °C, vorzugsweise bei +25 °C.

Die Einführung der Estergruppe 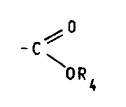
für R₁, bei welcher R₄ eine Alkylgruppe mit 1-10 C-Atomen darstellt, erfolgt nach den dem Fachmann bekannten Methoden. Die 1-Carboxy-verbindungen werden beispielsweise mit Diazokohlenwasserstoffen in an sich bekannter Weise umgesetzt. Die Veresterung mit Diazokohlenwasserstoffen erfolgt z. B. dadurch, daß man eine Lösung des Diazokohlenwasserstoffes in einem inerten Lösungsmittel, vorzugsweise in Diethylether, mit der 1-Carboxyverbindung in dem gleichen oder in einem anderen inerten Lösungsmittel, wie z. B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [org. Reactions Bd. 8, Seiten 389 - 394 (1954)].

Die Einführung der Estergruppe 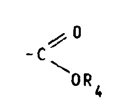
für R₁, bei welcher R₄ eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base, beispielsweise Pyridin, DMAP, Triethylamin, in einem inerten Lösungsmittel umgesetzt. Als Lösungsmittel kommen Methylenchlorid, Ethylenchlorid, Chloroform, Essigester, Tetrahydrofuran, vorzugsweise Chloroform in Frage. Die Reaktion wird bei Temperaturen zwischen -30 °C und +50 °C, vorzugsweise bei 10 °C, durchgeführt.

Sollen im Primärprodukt enthaltene C=C-Doppelbindungen reduziert werden, erfolgt die Hydrierung nach an sich bekannten Methoden.

Die Hydrierung des Δ^{8, 10}-Diensystems wird in an sich bekannter Weise bei tiefen Temperaturen, vorzugsweise bei etwa -20 °C bis +30 °C in einer Wasserstoffatmosphäre in Gegenwart eines Edelmetallkatalysators durchgeführt. Als Katalysator ist zum Beispiel 10 % Palladium auf Kohle geeignet.

Die Leukotrien-B₄-Derivate der Formel I mit R₄ in der Bedeutung eines Wasserstoffatoms können mit geeigneten Mengen der entsprechenden anorganischen Basen unter Neutralisierung in ein Salz überführt werden. Beispielsweise erhält man beim Lösen der entsprechenden Säuren in Wasser, das die stöchiometrische Menge der Base enthält, nach Abdampfen des Wassers oder nach Zugabe eines mit Wasser mischbaren Lösungsmittels, z.B. Alkohol oder Aceton, das feste anorganische Salz.

Zur Herstellung eines Aminsalzes wird die LTB₄-Säure z.B. in einem geeigneten Lösungsmittel, beispielsweise Ethanol, Aceton, Diethylether, Acetonitril oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Eine weitere Möglichkeit für die Einführung der Amidgruppen 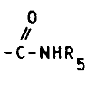
für R₁ besteht in der Umsetzung einer 1-Carbonsäure der Formel I (R₄=H), in der freie Hydroxygruppen gegebenenfalls intermediär geschützt sind, mit Verbindungen der Formel V,

O = C = N - R₅ (V),

worin R₅ die oben angegebene Bedeutung hat.

Die Umsetztung der Verbindung der Formel I (R₄=H) mit einem Isocyanat der Formel V erfolgt gegebenenfalls unter Zusatz eines tertiären Amins, wie z.B. Triethylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diethylether, Toluol, bei Temperaturen zwischen -80 °C bis 100 °C, vorzugsweise bei 0 °C bis 30 °C, vorgenommen werden.

Die Verbindungen der Formel I wirken antientzündlich und antiallergisch. Daneben besitzen sie antimykotische Eigenschaften. Folglich stellen die neuen Leukotrien B₄-Derivate der Formel I wertvolle pharmazeutische Wirkstoffe dar. Die Verbindungen der Formel I sind besonders zur topischen Applikation geeignet, da sie eine Dissoziation zwischen erwünschter topischer Wirksamkeit und unerwünschten systemischen Nebenwirkungen aufweisen.

Die neuen Leukotrien B₄-Derivate der Formel I eignen sich in Kombination mit den in der galenischen Pharmazie üblichen Hilfs- und Trägermitteln zur lokalen Behandlung von Kontaktdermatitis, Ekzemen der verschiedensten Art, Neurodermatosen, Erythrodermie, Verbrennungen, Tinea, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigeneten Zusätzen in die gewünschte Applikationsform, wie zum Beispiel: Lösungen, Lotionen, Salben, Cremes oder Pflaster, überführt, In den so formulierten Arzneimitteln ist die Wirkstoffkonzentration von der Applikationsform abhängig. Bei Lotionen und Salben wird vorzugsweise eine Wirkstoffkonzentration von 0,0001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Leukotrien-B₄-Derivate auch in Form von Kapseln, Tabletten oder Dragees, die vorzugsweise o,1 bis 100 mg Wirkstoff enthalten oder oral appliziert werden oder in Form von Suspensionen, die vorzugsweise 1-200 mg Wirkstoff pro Dosiseinheit enthalten und rektal appliziert werden auch zur Behandlung allergischer Erkrankungen des Darmtraktes, wie der Kolitis ulcerosa und der colitis granulomatosa.

Die neuen Leukotrien-B₄-Derivate können auch in Kombination, wie z.B mit Lipoxygenasehemmern, Cyclooxygenasehemmern, Prostacyclinagonisten, Thromboxanantagonisten, Leukotrien D₄-antagonisten, Leukotrien E₄-antagonisten, Leukotrien F₄-antagonisten, Phosphodiesterasehemmern oder PAF-Antagonisten verwendet werden.

### Beispiel 1

4-[2-[(1E,3E)-(5RS)-5-Hydroxy-1,3-tridecadienyl]-phenoxy]-buttersäureethylester
A. Zu einer Suspension von 4,36 g Natriumhydrid (55 %ige Dispersion in Mineralöl) in 80 ml Dimethylformamid wird unter Rühren und Eiskühlung innerhalb von 15 Minuten eine Lösung von 12,2 g Salicylaldehyd in 20 ml Dimethylformamid getropft. Nach 3 Stunden wird eine Lösung von 21,5 g 4-Brombuttersäureethylester in 16 ml Dimethylformamid innerhalb von 15 Minuten unter Eiskühlung zugetropft und die Mischung 72 Stunden bei Raumtemperatur gerührt. Der Ansatz wird auf Eis/Wasser gegossen, mit Ether ausgeschüttelt, die organische Phase kurz mit 1 n Natronlauge gewaschen, mit Natriumsulfat getrocknet und eingeengt. Das Rohprodukt wird bei 130-140 °C/0,1 mbar am Kugelrohr destilliert und man erhält so 11,2 g 4-(2-Formylphenoxy)-buttersäureethylester als farbloses Öl.
   IR: 2980, 2938, 2871, 1733, 1688, 1599, 1458, 1242, 1043, 760 cm⁻¹.
B. Zu einer Suspension von 7,4 g Chrom-II-chlorid in 100 ml Tetrahydrofuran wird bei 0 °C und Argonatmosphäre eine Lösung von 7,9 g Jodoform und 2,36 g 4-(2-Formylphenoxy)-buttersäureethylester in 50 ml Tetrahydrofuran getropft. Nach 2 1/2 Stunden wird die Mischung mit Wasser verdünnt, mit Diethylether ausgeschüttelt, die organische Phase mit Natriumsulfat getrocknet, eingeengt und der ölige Rückstand am Kieselgel mit Hexan/Ethylacetat 95/5 chromatographiert. Man erhält so 1,9 g eines gelben Öls, das zu 50 % aus 4-(2-[(E)-2-Jodvinyl]-phenoxy]-buttersäureethylester (I) und zu 50 % aus 4-[2-[(Z)-2-Jodvinyl]-phenoxy]-buttersäureethylester (II) besteht. Dieses E/Z-Gemisch wird an silanisiertem Kieselgel (RP-18 Material) mit Hilfe der Hochdruck-Flüssigkeits-Chromatographie getrennt.
   I: IR: 2978, 1732, 1595, 1451, 1245, 1179, 1102, 1049, 952, 750 cm⁻¹;
   II: IR: 2978, 1732, 1595, 1485, 1451, 1250, 1178, 1107, 1049, 752 cm⁻¹.
C. Analog Beispiel 1B werden 3,7 g Chrom-II-chlorid in 50 ml Tetrahydrofuran und eine Lösung von 3,95 g Jodoform und 1,18 g 4-(2-Formylphenoxy)-buttersäureethylester in 20 ml Tetraydrofuran zusammengegeben, mit 581 mg Tetramethylethylendiamin versetzt und die Mischung 6 Stunden unter Rückfluß erhitzt. Nach Aufarbeitung analog Beispiel 1B und Chromatographie an Kieselgel werden 660 mg gelbes Öl erhalten, das zu 70 % aus 4-[2-[(E)-2-Jodvinyl]-phenoxy]-buttersäureethylester und zu 30 % aus 4-[2-(Z-2-Jodvinyl)-phenoxy]buttersäureethylester besteht.
D. Eine Lösung von 154 mg 4-[2-[(E)-2-Jodvinyl]-phenoxy]-buttersäureethylester in 2 ml Dimethylformamid wird mit 6 mg Bis-(acetonitril)-palladium-II-chlorid versetzt und 10 Minuten bei Raumtemperautr gerührt. Danach werden 217 mg (E)-1-(Tri-n-butylstanmyl)-1-undecen-(3RS)-3-ol hinzugefügt und die Mischung 4 Stunden bei Raumtemperatur gerührt. Die Reaktionsmischung wird eingeengt und der Rückstand an Kieselgel mit n-Hexan/Ethylacetat = 85/15 chromatographiert. Es werden 100 mg 4-[2-[(1E,3E)-(5RS)-5-Hydroxy-1,3-tridecadienyl]-phenoxy]-buttersäureethylester als farbloses Öl erhalten.
   IR: 3460, 2925, 2857, 1735, 1600, 1490, 1455, 1245, 1180, 1050, 752 cm⁻¹.
   Die im Beispiel 1D verwendete Organozinnverbindung wird wie folgt hergestellt:
E. (E)-1-(Tri-n-butylstannyl)-1-undecen-(3RS)-3-ol
   a) Zu einer Lösung von 25 g Trimethylsilylacetylen in 500 ml Tetrahydrofuran werden unter Rühren und Argonatmosphäre bei -40 °C 173 ml n-Butyllithiumlösung (1,6 molar in Hexan) getropft, 30 Minuten bei -40 °C gerührt, auf -70 °C gekühlt und 48,1 ml Nonanal bei dieser Temperatur zugetropft. Nach einer Stunde bei -70 °C, läßt man die Mischung auf -30 °C kommen, fügt 300 ml 2n Salzsäure hinzu und schüttelt mit Diethylether aus. Die organischen Phasen werden getrocknet (Na₂SO₄), eingeengt und der Rückstand am Kugelrohr bei 120-130 °C/0,2 mbar destilliert. Man erhält so 48,5 (3RS)-1-(Trimethylsilyl)-1-undecin-3-ol als farbloses Öl.
      IR: 3350, 2965, 2930, 2860, 2178, 1250, 843, 760 cm ⁻¹.
   b) In einer Mischung aus 420 ml 0,1 n Natronlauge und 420 ml Methanol werden 10 g (3RS)-1-(Trimethylsilyl)-1-undecin-3-ol gelöst und 3 Stunden bei Raumtemperatur gerührt. Anschließend wird das Methanol im Vakuum entfernt, mit Diethylether ausgeschüttelt, getrocknet (Na₂SO₄) und eingeengt. Man erhält 6,46 g (3RS)-1-Undecin-3-ol als gelbes Öl, das als Rohprodukt weiter umgesetzt wird.
      IR: 3318, 2925, 2855, 1680, 1467, 1460, 1380, 1030, 655, 627 cm⁻¹.
   c) Eine Mischung aus 3 g (3RS)-1-Undecin-3-ol, 7,12 ml Tri-n-butylzinnhydrid und 36 ml 2,2'-Azoisobuttersäurenitril wird unter Argonatmosphäre 2 Stunden auf 80 °C erhitzt. Das Reaktionsgemisch wird an Kieselgel, das mit 2 % (Gewichtsprozente) Triethylamin desaktiviert ist, mit n-Hexan chromatographiert. Es werden 4,4 g (E)-1-Tri-n-butylstannyl)-1-undecen-(3RS)-3-ol als farbloses Öl erhalten.
      IR: 3340, 2958, 2929, 2858, 1465, 1377, 1072, 990 cm⁻¹.

### Beispiel 2

4-[2-[(1E,3E)-(5RS)-5-Hydroxy-1,3-tridecadienyl]-phenoxy]-buttersaureethylester
A. 8,65 g Bromphenol und 8,75 g 4-Brombuttersäureethylester werden in 120 ml Dimethylformamid gelöst, mit 32,6 g Cäsiumcarbonat versetzt und 2 Tage bei Raumtemperatur gerührt. Die Reaktionsmischung wird filtriert, das Lösungsmittel im Vakuum abdestilliert und der Rückstand am Kugelrohr bei 180 °C/ 0,4 mbar destilliert. Es werden 11 g 4-(2-Bromphenoxy)-buttersäureethylester als farbloses Öl erhalten.
   IR: 2980, 1730, 1590, 1469, 1278, 1250, 1180, 1053, 1030, 750 cm⁻¹.
B. Unter den Bedingungen des Beispiels 2A werden 25 g 2-Jodphenol und 22,16 g 4-Brombuttersäureethylester in 100 ml Dimethylformamid in Gegenwart von 74 g Cäsiumcarbonat umgesetzt, aufbereitet und man erhält 24,2 g 4-(2-Jodphenoxy)-buttersäureethylester als farbloses Öl vom Siedepunkt 200 °C/ 0,02 mbar.
   IR: 2980, 1730, 1584, 1465, 1275, 1246, 1180, 1050, 1017, 749 cm⁻¹.
C. 861 mg 4-(2-Bromphenoxy)-buttersäureethylester und 4,8 g (E)-1,2-Bis-(tri-n-butylstannyl)-ethylen (50 %ig) werden in 11 ml Toluol gelöst, mit 70 mg Tetrakis-(triphenylphosphin)-palladium versetzt und unter Rühren und Argonatmosphäre 1 Stunde auf 120 °C (Badtemperatur) erhitzt. Nach Abkühlen auf Raumtemperatur wird die Reaktionsmischung mit 9 ml Diethylether verdünnt und bei 0 °C mit einer Lösung von 1,53 g Jod in 7 ml Diethylether versetzt. Man rührt die Mischung 2 Stunden bei 0 °C und läßt sie über Nacht im Kühlschrank stehen. Der Ansatz wird eingeengt und der Rückstand an Kieselgel mit n-Hexan/Ethylacetat = 95/5 chromatographiert. Es werden 810 mg 4-[2-[(E)-2-Jodvinyl]-phenoxy]-buttersäureethylester als öliges Rohprodukt erhalten, das ohne weitere Reinigung weiter umgesetzt wird.
D. 1,34 g (4-(2-Jodphenoxy)-buttersäureethylester und 6,4 g (E)-1,2-Bis-(tri-n-butylstannyl)-ethylen (50 %ig) werden in 12 ml Toluol gelöst, mit 94 mg Tetrakis-(triphenylphosphin)-palladium versetzt und unter Rühren und Argonatmosphäre 2 Stunden auf 70-80 °C (Badtemperatur) erhitzt. Nach Abkühlung auf Raumtemperatur wird die Reaktionsmischung mit 12 ml Diethylether verdünnt und unter den Bedingungen des Beispiels 2C mit einer Lösung von 2,04 g Jod in 10 ml Diethylether behandelt, aufbereitet und an Kieselgel mit n-Hexan/Ethylacetat = 95/5 chromatographiert. Es werden 900 mg 4-[-[(E)-2-Jodvinyl]-phenoxy]-buttersäureethylester als gelbes Öl erhalten.
   IR: siehe Beispiel 1B:I
E. Unter den Bedingungen des Beispiels 1D werden 400 mg des nach Beispiel 2C oder 2D erhaltenen 4-[-[(E)-2-Jodvinyl]-phenoxy]-buttersäureethylesters in 2 ml Dimethylformamid in Gegenwart von 36 mg 1,1'-Bis-(diphenylphosphino)-ferrocen-palladium-II-chlorid mit 460 mg (E)-1-(Tri-n-butylstannyl)-1-undecen-(3RS)-3-ol ungesetzt, aufbereitet und an Kieselgel mit n-Hexan/Ethyl-acetat = 9/1 chromatographiert. Es werden 160 mg 4-[2-[(1E,3E)-(5RS)-5-Hydroxy-1,3-tridecadienyl]-phenoxy]-buttersäureethylester als farbloses Öl erhalten.
   IR: siehe Beispiel 1D.

### Beispiel 3

4-[2-[(1E,3E)-(5R)-5-Hydroxy-1,3-tridecadienyl]-phenoxy]-buttersäureethylester
A. Eine Mischung aus 917 mg (E)-1-(Tri-n-butylstannyl)-1-undecen-(3RS)-3-ol, 112,6 mg L-(+)-Weinsäurediisopropylester und 275 mg gepulvertes Molsieb (3Å) in 4 ml Methylenchlorid wird bei -15 °C mit 113,7 mg Titan-IV-isopropylat versetzt und 20 Minuten bei -20 °C gerührt. Danach wird bei -20 °C 1 ml tert.-Butylhydroperoxid (3 molar in Isooctan) hinzugefügt und die Mischung 4 Stunden bei dieser Temperatur gerührt. Der Ansatz wird bei 0 °C mit einer Lösung aus 3,3 g Eisen-II-sulfat und 1 g Weinsäure in 10 ml Wasser versetzt und 10 Minuten bei 0 °C gerührt. Danach wird mit Diethylether ausgeschüttelt, die organische Phase getrocknet (Na₂SO₄) eingeengt und das Rohprodukt an Kieselgel mit n-Pentan/Diethylether = 95/5 chromatographiert. Es werden 245 mg (E)-1-(Tri-n-butylstannyl)-1-undecen-(3R)-3-ol als farbloses Öl erhalten.
   [α]_{D} = - 9,3 ° (2 % in Ethanol).
   IR: siehe Beispiel 1E,c.
B. Unter den Bedingungen des Beispiels 1D werden 115 mg 4-[2-[(E)-2-Jodvinyl)-phenoxy]-buttersäureethylester in 2 ml Dimethylformamid in Gegenwart von 11,5 g 1,1'-Bis-(diphenylphosphino)-ferrocen-palladium-II-chlorid mit 147 mg (E)-1-(Tri-n-Butylstannyl)-1-undecen-(3R)-3-ol umgesetzt, aufbereitet und an Kieselgel mit n-Hexan/Ethylacetat = 9/1 chromatographiert. Es werden 55 mg 4-[2-[(1E,3E)-(5R)-5-Hydroxy-1,3-tridecadienyl]-phenoxy]-buttersäureethyl-ester als farbloses Öl erhalten.
   [α]_{D} = - 12 ° (5 % in Ethanol).
   IR: siehe Beispiel 1D.
C. 10 mg 4-[2-[(1E,3E)-(5R)-5-Hydroxy-1,3-tridecadienyl]-phenoxy]-buttersäureethylester werden durch Racemattrennung von 50 mg 4-[2-[(1E,3E)-(5RS)-5-Hydroxy-1,3-tridecadienyl]-phenoxy]-buttersäureethylester an einer Chiralcel OD-Säule mit Hilfe der Hochdruck-Flüssigkeits-Chromatographie und n-Hexan/ Isopropanol = 8/2 als Elutionsmittel gewonnen.
   [α]_{D} = - 12,6 ° (5 % in Ethanol)

### Beispiel 4

4-[2-[(1Z,3E)-(5RS)-5-Hydroxy-1,3-tridecadienyl]-phenoxy]-buttersäureethylester

Unter den Bedingungen des Beispiels 1D werden 180 mg 4-[2-[(Z)-2-Jodvinyl]-phenoxy]-buttersäureethylester in 2 ml Dimethylformamid in Gegenwart von 7 mg Bis-(acetonitril)-palladium-II-chlorid mit 254 mg (E)-1-(Tri-n-butylstannyl)-1-undecen-(3RS)-3-ol umgesetzt, aufbereitet und an Kieselgel mit n-Hexan/Ethylacetat = 98/2 chromatographiert. Es werden 95 mg 4-[2-[(1Z,3E)-(5RS)-5-Hydroxy-1,3-tridecadienyl]-phenoxy]-buttersäureethylester als farbloses Öl erhalten.
IR: 3460, 2922, 2850, 1735, 1597, 1488, 1401, 1245, 1178, 1050, 750 cm⁻¹.

### Beispiel 5

4-[2-[(1E,3E)-(5RS)-5-Hydroxy-1,3-tridecadienyl]-phenoxy]-buttersäuremethylester
A. Eine Lösung von 2 g 4-(2-Bromphenoxy)-buttersäureethylester in 20 ml Methanol und 20 ml 1n Natronlauge wird 2 Stunden bei Raumtemperatur gerührt, danach mit 0,1 n Salzsäure angesäuert und mit Diethylether extrahiert. Die organische Phase wird getrocknet (Na₂SO₄), eingeengt und man erhält 1,4 g 4-(2-Bromphenoxy)-buttersäure vom Schmelzpunkt 80-81 °C.
   IR: 3518, 3060, 2960, 1710, 1588, 1467, 1442, 1275, 1245, 1125, 1050, 1030, 952 cm⁻¹.
B. Eine Lösung von 1,4 g 4-(2-Bromphenoxy)-buttersäure in 22 ml Methanol wird mit 1g Amberlyst 15 versetzt und 5 Stunden bei Raumtemperatur gerührt. Danach wird vom Amberlyst 15 abfiltriert, das Filtrat eingeengt, der Rückstand in Diethylether aufgenommen, mit verdünnter Natriumcarbonatlösung gewaschen, getrocknet (Na₂SO₄) und eingeengt. Es werden 1,5 g 4-(2-Bromphenoxy)-buttersäuremethylester als farbloses Öl erhalten.
   IR: 2970, 1735, 1590, 1483, 1470, 1442, 1278, 1249, 1175, 1053, 1030, 750 cm⁻¹.
C. Unter den Bedingungen des Beispiels 2C werden 482 mg 4-(2-Bromphenoxy)-buttersäuremethylester und 3,2 g (E)-1,2-Bis-(tri-n-butylstannyl)-ethylen (50 %ig) in 6 ml Toluol in Gegenwart von 47 mg Tetrakis-(triphenylphosphin)-palladium erhitzt, mit 6 ml Diethylether verdünnt, mit einer Lösung von 1,02 g Jod in 5 ml Diethylether versetzt, eingeengt und der Rückstand an Kieselgel chromatographiert. Es werden 400 mg 4-[2-[(E)-2-Jodvinyl]-phenoxy]-buttersäuremethylester als öliges Rohprodukt erhalten, das ohne weitere Reinigung in der nächsten Stufe eingesetzt wird.
D. Unter den Bedingungen des Beispiels 1D werden 400 mg 4-[-2-[(E)-2-Jodvinyl]-phenoxy]-buttersäuremethylester in Gegenwart von 16,2 mg Bis-(acetonitril)-palladium-II-chlorid mit 599 mg (E)-1-(Tri-n-Butylstannyl)-1-undecen-(3RS)-3-ol umgesetzt, aufbereitet und chromatographiert. Es werden 125 mg 4-[2-[(1E,3E)-(5RS)-5-Hydroxy-1,3-tridecadienyl]-phenoxy]-buttersäuremethylester als farbloses Öl erhalten, das zur vollständigen Reinigung der Hochdruck-Flüssigkeits-Chromatographie an silanisiertem Kieselgel (RP 18-Material) mit Methanol/Wasser = 8/2 unterworfen wird.
   IR: 3430, 2925, 2855, 1738, 1595, 1490, 1455, 1242, 1171, 1050, 971, 749 cm⁻¹.

### Beispiel 6

4-(2-[(1E,3E)-(5RS)-5-Hydroxy-1,3-tridecadienyl]-phenoxy]-buttersäureisopropylester
A. Unter den Bedingungen des Beispiels 5A wird 1 g 4-(2-Jodphenoxy)-buttersäureethylester in 10 ml Methanol und 10 ml 1n Natronlauge verseift, aufbereitet und man erhält 900 mg 4-(2-Jodphenoxy)-buttersäuremethylester vom Schmelzpunkt 66,5-67 °C.
   IR: 3510, 2940, 2670, 1710, 1585, 1467, 1440, 1278, 1050, 1018, 953, 648 cm⁻¹.
B. Eine Lösung von 4-(2-Jodphenoxy)-buttersäure in 10 ml Isopropanol wird mit 0,5 g Amberlyst 15 versetzt, über Nacht bei Raumtemperatur gerührt und anschließend 2 Stunden unter Rückfluß erhitzt. Danach wird vom Amberlyst 15 abfiltriert, das Filtrat eingeengt, der Rückstand in Diethylether aufgenommen, mit verdünnter Natriumcarbonatlösung gewaschen, getrocknet und eingeengt. Es werden 920 mg 4-(2-Jodphenoxy)-buttersäureisopropylester als farbloses Öl erhalten.
   IR: 2962, 2938, 1718, 1584, 1465, 1438, 1325, 1275, 1260, 1245, 1102. 1015, 943, 646 cm⁻¹.
C. Unter den Bedingungen des Beispiels 2D werden 696 mg 4-(2-Jodphenoxy)-buttersäureisopropylester und 3,2 g (E)-1,2-Bis-(tri-n-butylstannyl)-ethylen (50 %ig) in Gegenwart von 47 mg Tetrakis-(triphenylphosphin)-palladium umgesetzt, mit 1,02 g Jod behandelt, aufbereitet und an Kieselgel mit n-Hexan/ Ethylacetat 9/1 chromatographiert. Es werden 340 mg 4-[-2-[(E)-2-Jodvinyl]-phenoxy]-buttersäurepropylester als öliges Rohprodukt erhalten, das ohne weitere Reinigung in der nächsten Stufe eingesetzt wird.
D. Unter den Bedingungen des Beispiels 1D werden 330 mg 4-[-2-[(E)-2-Jodvinyl]-phenoxy]-buttersäureisopropylester in Gegenwart von 32 mg 1,1'-Bis-(diphenylphosphino)-ferrocen-palladium-II-chlorid mit 445 mg (E)-1-(Tri-n-butylstannyl)-1-undecen-(3RS)-3-ol ungesetzt, aufbereitet und an Kieselgel mit n-Hexan/Ethylacetat = 95/5 chromatographiert. Es werden 189 mg 4-[2-[(1E,-3E)-(5RS)-5-Hydroxy-1,3-tridecadienyl]-phenoxy]-buttersäureisopropylester als farbloses Öl erhalten, das zur vollständigen Reinigung der Hochdruck-Flüssigkeits-Chromatographie an silanisiertem Kieselgel (RP 18-Material) mit Methanol/Wasser = 8/2 unterworfen wird.
   IR: 2928, 2860, 1722, 1600, 1455, 1378, 1260, 1104 cm⁻¹.

### Beispiel 7

4-[2-[(1E,3E)-(5RS)-5-Acetoxy-1,3-tridecadienyl]-phenoxy]-buttersäureethylester

Eine Lösung von 60 mg 4-[2-[(1E,3E)-(5RS)-5-Hydroxy-1,3-tridecadienyl]-phenoxy]-buttersäureethylester in 0,5 ml Pyridin wird unter Eiskühlung und Argonatmosphäre mit 0,12 ml Acetanhydrid versetzt und 4 Stunden bei Raumtemperatur gerührt. Die Reaktionsmischung wird eingeengt und der Rückstand an Kieselgel mit n-Hexan/Ethylacetat = 95/5 chromatographiert. Es werden 42 mg 4-[2-[(1E,-3E)-(5RS)-5-Acetoxy-1,3-tridecadienyl]-phenoxy]-buttersäureethylester als farbloses Öl erhalten.
IR: 3928, 2858, 1735, 1596, 1490, 1455, 1372, 1242, 992, 750 cm⁻¹.

### Beispiel 8

4-[(5RS)-5-Hydroxy-tridecyl]-phenoxy]-buttersäureethylester
A. Unter den Bedingungen des Beispiels 1D werden 850 mg 4-[-2-[(E/Z)-2-Jodvinyl]-phenoxy]-buttersäureethylester in Gegenwart von 85 mg 1,1'-Bis-(diphenylphosphino)-ferrocen-palladium-II-chlorid mit 1,3 g (1E)-1-(Tri-n-butyl-stannyl)-1-undecen-(3RS)-3-ol umgesetzt, aufbereitet und an Kieselgel mit n-Hexan/Ethylacetat = 9/1 chromatographiert. Es werden 800 mg 4-[2-[(1E/Z,-3E)-(5RS)-5-Hydroxy-1,3-tridecadienyl]-phenoxy]-buttersäureethylester als farbloses Öl erhalten.
B. Eine Lösung von 400 mg 4-[2-[(1E/Z,3E)-(5RS)-5-Hydroxy-1,3-tridecadienyl]-phenoxy]-buttersäureethylester wird in Gegenwart von 40 mg 10 %igem Palladium-Katalysator auf Aktivkohle unter Schütteln 15 Minuten bei Raumtemperatur und Normaldurck hydriert. Die Reaktionslösung wird filtriert, eingeengt und an Kieselgel mit n-Hexan/Ethylacetat = 9/1 chromatographiert. Es werden 290 mg 4-[2-[(5RS)-5-Hydroxy-tridecyl]-phenoxy]-buttersäureethylester als farbloses Öl erhalten, das zur vollständigen Reinigung der Hochdruck-Flüssigkeits-Chromatographie an silanisiertem Kieselgel (RP 18-Material) mit Methanol/Wasser = 8/2 unterworfen wird.
   IR: 3440, 2928, 2860, 1725, 1600, 1595, 1455, 1375, 1260, 1095 cm⁻¹.

### Beispeil 9

4-[2-[(1E,3E)-(5RS)-5-Hydroxy-1,3-tridecadienyl]-phenoxy]-buttersäure

Eine Lösung von 130 mg 4-[2-[(1E,3E)-(5RS)-5-Hydroxy-1,3-tridecadienyl]-phenoxy]-buttersäureethylester in 10 ml Methanol und 2,5 ml 0,5 n Natronlauge wird unter Argonatmosphäre 3 Stunden bei Raumtemperatur gerührt. Die Reaktionsmischung wird auf Eiswasser gegossen, mit 0,5 n Schwefelsäure auf pH 5 angesäuert und mit Ethylacetat ausgeschüttelt. Die organische Phase wird getrocknet, eingeengt und der Rückstand an Kieselgel mit n-Hexan/Diethylether = 7/3 chromatographiert. Es werden 78 mg der Titelverbindunge als farbloses Öl erhalten.
IR: 3437, 2920, 2851, 1709, 1594, 1488, 1453, 1240, 1100, 1048, 990, 802, 746 cm⁻¹.

### Beispiel 10

4-[2-[(1Z,3E)-(5RS)-5-Hydroxy-1,3-tridecadienyl]-phenoxy]-buttersäure

Unter den Bedingungen des Beispiels 9 wird eine Lösung von 60 mg 4 [2 [1Z, 3E)-(5RS)-5-Hydroxy-1,3-tridecadienyl]-phenoxy]-buttersäureethylester in 5 ml Methanol und 2 ml 0,5 n Natronlauge verseift, aufbereitet und an Kieselgel mit n-Hexan/Ethylacetat = 98/2 chromatographiert. Es werden 55 mg der Titelverbindung als farbloses Öl erhalten.
IR: 3436, 2928, 2855, 1712, 1594, 1488, 1450, 1244, 751 cm⁻¹.

### Beispiel 11

4-[2-[(1E,3E)-(5RS)-5-Hydroxy-1,3-tridecadienyl]-phenoxy]-butan-1-ol
A. Zu einer Suspension von 80 mg Lithiumaluminiumhydrid in 5 ml Tetrahydrofuran wird bei 0 °C unter Rühren und Argonatmosphäre eine Lösung von 250 mg 4-[2-[(E)-2-Jodvinyl]-phenoxy]-buttersäureethylester in 5 ml Tetrahydrofuran getropft. Danach wird eine Stunde bei Eiskühlung gerührt, die Mischung unter Argonatmosphäre mit Eiswasser zersetzt, mit Diethylether ausgeschüttelt, getrocknet (Na₂SO₄) und eingeengt. Es werden 120 mg 4-[2-[(E)-2-Jodvinyl]-phenoxy]-butan-1-ol als gelbes Öl erhalten, das als Rohprodukt weiter umgesetzt wird.
B. Unter den Bedingungen des Beispiels 1D werden 110 mg 4-[2-[(E)-2-Jodvinyl]-phenoxy]-butan-1-ol in Gegenwart von 13 mg 1,1'-Bis-(diphenylphosphino)-ferrocen-palladium-II-chlorid mit 200 mg (E)-1-(Tri-n-butylstannyl)-1-undecen-(3RS)-3-ol umgesetzt, aufbereitet und an Kieselgel mit n-Hexan/Ethylacetat = 8/2 chromatographiert. Es werden 110 mg 4-[2-[(1E,3E)-(5RS)-5-Hydroxy-1,3-tridecadienyl]-phenoxy]-butan-1-ol als farbloses Öl erhalten.
   IR: 3355, 2925, 2855, 1594, 1487, 1453, 1242, 1100, 1048, 995, 748 cm⁻¹.

### Beispiel 12

2-[2-[(1E,3E)-(5RS)-5-Hydroxy-1,3-tridecadienyl]-phenoxy]-essigsäureethylester
A. Unter den Bedingungen des Beispiels 2A werden 3 g 2-Jodphenol in Gegenwart von 8,9 g Cäsiumcarbonat mit 2,27 g Bromessigsäureethylester umgesetzt, aufbereitet und man erhält 1,7 g 2-(2-Jodphenoxy)-essigsäureethylester als farbloses Öl vom Siedepunkt 170 °C/0,4 mbar.
   IR: 3065, 2980, 2935, 1740, 1583, 1470, 1445, 1377, 1290, 1200, 1122, 1075, 930, 855, 792, 750, 645 cm⁻¹.
B. Unter den Bedingungen des Beispiels 2D werden 612 mg 2-(2-Jodphenoxy)-essigsäureethylester und 3,2 g (E)-1,2-Bis-(tri-n-butylstannyl)-ethylen (50 %ig) in Gegenwart von 47 mg Tetrakis-(triphenylphosphin)-palladium umgesetzt, mit 1,02 g Jod behandelt, aufbereitet und an Kieselgel mit n-Hexan/Ethylacetat = 95/5 chromatographiert. Es werden 280 mg 2-[2-[(E)-2 Jodvinyl]phenoxy]-essigsäureethylester als öliges Rohprodukt erhalten, das ohne weitere Reinigung in der nächsten Stufe eingesetzt wird.
C. Unter den Bedingungen des Beispiels 1D werden 270 mg 2-[2-[(E)-2-Jodvinyl]-phenoxy]-essigsäureethylester in Gegenwart von 29 mg 1,1'-Bis-(diphenylphosphino)-ferrocen-palladium-II-chlorid mit 410 mg (E)-1-(Tri-n-butylstannyl)-1-undecen-(3RS)-3-ol umgesetzt, aufbereitet und an Kieselgel n-Hexan/Ethylacetat = 9/1 chromatographiert. Es werden 200 mg 2-[2-[(1E,3E)-(5RS)-5-Hydroxy-1,3-tridecadienyl]-phenoxy]-essigsäureethylester als farbloses Öl erhalten, das zu vollständigen Reinigung der Hochdruck-Flüssigkeits-Chromatographie an silanisiertem Kieselgel (RP 18-Material) mit Methanol/Wasser 85/15 unterworfen wird.
   IR: 2960, 2928, 2855, 1755, 1600, 1485, 1260, 1095, 1010 cm⁻¹.

### Beispiel 13

2-[2-[(1E,3E)-(5RS)-5-Hydroxy-1,3-tridecadienyl]-phenoxy]-essigsäure

Unter den Bedingungen des Beispiels 9 werden 130 mg 2-[2-[(1E,3E)-(5RS)-5-Hydroxy-1,3-tridecadienyl]-phenoxy]-esssigsäureethylester verseift, aufbereitet und das Rohprodukt mit Hilfe der Hochdruck-Flüssigkeits-Chromatographie an silanisiertem Kieselgel (RP 18-Material) mit Methanol/Wasser/Essigsäure = 8/2 2 %₁ gereinigt. Es werden 25 mg der Titelverbindung als farbloses Öl erhalten.
IR: 3690, 2960, 2928, 2855, 1704, 1260, 1098, 1013 cm⁻¹.

### Beispiel 14

5-[2-[(1E,3E)-(5RS)-5-Hydroxy-1,3-tridecadienyl]-phenoxy]-valeriansäureethylester
A. Unter den Bedingungen des Beispiels 2A werden 3 g 2-Jodphenol in Gegenwart von 8,9 g Cäsiumcarbonat mit 2,84 g 5-Bromvaleriansäureethylester umgesetzt, aufbereitet und man erhält 1,8 g 5-(2-Jodphenoxy)-valeriansäureethylester als farbloses Öl vom Siedepunkt 210 °C/0,4 mbar.
   IR: 3060, 2980, 2940, 2870, 1730, 1584, 1467, 1438, 1278, 1248, 1162, 1050, 1018, 750, 650 cm⁻¹.
B. Unter den Bedingungen des Beispiels 2D werden 696 mg 5-(2-Jodphenoxy)-valeriansäureethylester und 3,2 g (E)-1,2-Bis-(tri-n-butylstannyl)-ethylen (50 %ig) in Gegenwart von 47 mg Tetrakis-(triphenylphosphin)-palladium umgesetzt, mit 1,02 g Jod behandelt, aufbereitet und an Kieselgel mit n-Hexan/ Ethylacetat = 95/5 chromatographiert. Es werden 430 mg 5-[2-[(E)-2-Jodvinyl]-phenoxy]-valeriansäureethylester als öliges Rohprodukt erhalten, das ohne weitere Reinigung in der nächsten Stufe eingesetzt wird.
C. Unter den Bedingungen des Beispiels 1D werden 400 mg 5-[2-[(E)-2-Jodvinyl]-phenoxy]-valeriansäureethylester in Gegenwart von 39 mg 1,1'-Bis-(diphenylphosphino)-ferrocen-palladium-II-chlorid mit 540 mg (E)-1-(Tri-n-butylstannyl)-1-undecen-(3RS)-3-ol umgesetzt, aufbereitet und an Kieselgel mit n-Hexan/Ethylacetat = 98/2 chromatographiert. Es werden 257 mg 5-[2-[(1E, 3E)-(5RS)-5-Hydroxy-1,3-tridecadienyl]-phenoxy]-valeriansäureethylester als farbloses Öl erhalten.
   IR: 3500, 2925, 2858, 1723, 1600, 1453, 1375, 1240, 1162, 1100, 992 cm⁻¹.

### Beispiel 15

4-[2-[(1E,3E)-(5RS)-5-Hydroxy-1,3-tridecadienyl]-phenoxy]-buttersäure-tetramethylenamid
A. Eine Lösung von 2 g 4-(2-Jodphenoxy)-buttersäureethylester und 200 mg Ammoniumchlorid in 6 ml Pyrrolidin wird 2 Stunden unter Rühren und Rückfluß erhitzt. Der Ansatz wird in Wasser gegossen, mit Diethylether ausgeschüttelt, die organische Phase mit 2 n Salzsäure und Wasser gewaschen, getrocknet und eingeengt. Der Rückstand wird bei 210 °C/0,4 mbar am Kugelrohr destilliert und man erhält 1,6 g 4-(2-Jodphenoxy)-buttersäure-tetramethylenamid als farbloses Öl.
   IR: 2970, 2875, 1637, 1583, 1462, 1440, 1276, 1247, 1048, 1015, 748 cm⁻¹.
B. Unter den Bedingungen des Beispiels 2D werden 718 mg 4-(2-Jodphenoxy)-buttersäuretetramethylenamid und 3,2 g (E)-1,2-Bis-(tri-n-butylstannyl)-ethylen (50 %ig) in Gegenwart von 47 mg Tetrakis-(triphenylphosphin)-palladium umgesetzt, mit 1,02 g Jod behandelt, aufbereitet und an Kieselgel mit n-Hexan/ Ethylacetat = 8/2 chromatographiert. Es werden 450 mg 4-[2-[(E)-2-Jodvinyl]-phenoxy]-buttersäure-tetramethylenamid als öliges Rohprodukt erhalten, das ohne weitere Reinigung in der nächsten Stufe eingesetzt wird.
C. Unter den Bedingungen des Beispiels 1D werden 440 mg 4-[2-[(E)-2-Jodvinyl]-phenoxy]-buttersäure-tetramethylenamid in Gegenwart von 42 mg 1,1'-Bis-(diphenylphosphino)-ferrocen-palladium-II-chlorid mit 670 mg (E)-1-(Tri-n-butylstannyl)-1-undecen-(3RS)-3-ol umgesetzt, aufbereitet und an Kieselgel mit n-Hexan/Ethylacetat = 7/3 chromatographiert. Es werden 150 mg 4-[2-[(1E,3E)-(5RS)-5-Hydroxy-1,3-tridecadienyl]-phenoxy]-buttersäure-tetramethylenamid als farbloses Öl erhalten.
   IR: 2928, 2857, 1625, 1450, 1100, 953 cm⁻¹.

## Patentansprüche

1. Leukotrien-B₄-Derivate der Formel I 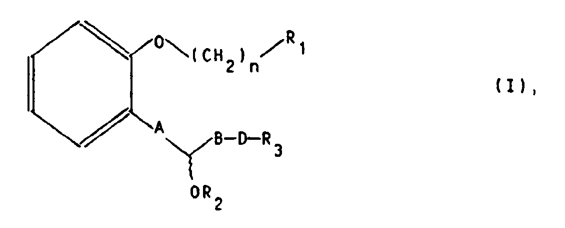 worin n = 1-10,
R₁ den Rest CH₂OH, den Rest COOR₄ mit R₄ in der Bedeutung eines Wasserstoffatoms, eines Alkylrestes mit 1-10 C-Atomen, eines Cycloalkylrestes mit 3-10 C-Atomen, eines gegebenenfalls durch 1-2 Chlor, Brom, Phenyl, Alkyl mit 1-4 C-Atomen, Chlormethyl, Fluormethyl, Trifluormethyl, Carboxy, C₁-C₄-Alkoxy oder Hydroxy substituierten Arylrestes mit 6-10 C-Atomen, eines -CH₂-CO-Aryl-Restes mit 6-10 C-Atomen für Aryl oder eines 5-6-gliedrigen heterocyclischen Restes mit wenigstens 1 Heteroatom, den Rest CONHR₅ mit R₅ in der Bedeutung eines Alkanoyl- oder Alkansulfonylrestes mit 1-10 C-Atomen oder des Restes R₄ oder den Rest CONR₆R₇, wobei R₆ und R₇ Alkyl mit 1-10 C-Atomen oder gemeinsam einen Alkylenrest mit 3-6 C-Atomen bedeuten,
A eine cis, trans- oder trans, trans-CH=CH-CH=CH- oder Tetramethylengruppe,
B eine geradkettige oder verzweigtkettige gesättigte oder ungesättigte Alkylengruppe mit bis zu 10 C-Atomen, die gegebenenfalls durch Fluor substituiert sein kann, oder die Gruppe 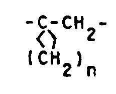 mit n = 1, 2 oder 3,
D eine Direktverbindung, Sauerstoff, Schwefel, eine -C≡C-Gruppe oder eine -CH=CR₇-Gruppe mit R₇ als Wasserstoff, C₁-C₅-Alkyl, Chlor oder Brom,
B und D gemeinsam eine direkte Bindung,
R₂ ein Wasserstoffatom oder einen Säurerest einer organischen Säure mit 1-15 C-Atomen und
R₃ ein Wasserstoffatom, einen Alkylrest mit 1-10 C-Atomen, einen durch Chlor oder Brom substituierten Alkylrest mit 1-10 C-Atomen, einen Cycloalkylrest mit 3-10 C-Atomen, einen gegebenenfalls durch 1-2 Chlor-, Brom, Phenyl, Alkyl mit 1-4 C-Atomen, Chlormethyl, Fluormethyl, Trifluormethyl, Carboxy, C₁-C₄-Alkoxy oder Hydroxy substituierten Arylrest mit 6-10 - C-Atomen oder einen 5-6-gliedrigen heterocyclischen Rest mit wenigstens 1 Heteroatom bedeuten und, falls R₄ die Bedeutung eines Wasserstoffatom hat, deren Salze mit physiologisch verträglichen Basen und deren Cyclodextrinclathrate.

2. 4-[2-[(1E,3E)-(5RS)-5-Hydroxy-1,3-tridecadienyl]-phenoxy]-buttersäureethylester

3. 4-[2-[(1E,3E)-(5R)-5-Hydroxy-1,3-tridecadienyl]-phenoxy]-buttersäureethylester

4. 4-[2-[(12,3E)-(5RS)-5-Hydroxy-1,3-tridecadienyl]-phenoxy]-buttersäureethylester

5. 4-[2-[(1E,3E)-(5RS)-5-Hydroxy-1,3-tridecadienyl]-phenoxy]-buttersäuremethylester

6. 4-[2-[(1E,3E)-(5RS)-5-Hydroxy-1,3-tridecadienyl]-phenoxy]-buttersäureisopropylester

7. 4-[2-[(1E,3E)-(5RS)-5-Acetoxy-1,3-tridecadienyl]-phenoxy]-buttersäureethylester

8. 4-[(5RS)-5-Hydroxy-tridecyl]-phenoxy]-buttersäureethylester

9. 4-[2-[(1E,3E)-(5RS)-5-Hydroxy-1,3-tridecadienyl]-phenoxy]-buttersäure

10. 4-[2-[(1Z,3E)-(5RS)-5-Hydroxy-1,3-tridecadienyl]-phenoxy]-buttersäure

11. 4-[2-[(1E,3E)-(5RS)-5-Hydroxy-1,3-tridecadienyl]-phenoxy]-butan-1-ol

12. 2-[2-[(1E,3E)-(5RS)-5-Hydroxy-1,3-tridecadienyl]-phenoxy]-essigsäureethylester

13. 2-[2-[(1E,3E)-(5RS)-5-Hydroxy-1,3-tridecadienyl]-phenoxy]-essigsäure

14. 5-[2-[(1E,3E)-(5RS)-5-Hydroxy-1,3-tridecadienyl]-phenoxy]-valeriansäure ethylester

15. 4-[2-[(1E,3E)-(5RS)-5-Hydroxy-1,3-tridecadienyl]-phenoxy]-buttersäure-tetramethylenamid

16. Verfahren zur Herstellung der Leukotrien-B₄-Derivate der Formel I, gemäß Anspruch 1 dadurch gekennzeichnet, daß man ein Vinylhalogenid der Formel II oder III, 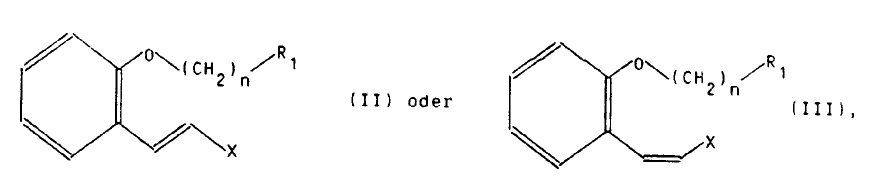 worin R₁ und n die bereits angegebenen Bedeutungen haben und X ein Jod- oder Bromatom ist, mit einer zinnorganischen Verbindung der Formel IV, 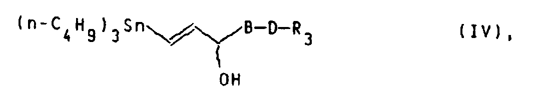 worin B, D und R₃ die bereits angegebenen Bedeutungen haben, mit Hilfe eines Palladium-Katalysators umsetzt und gegebenenfalls anschließend in beliebiger Reihenfolge Enantiomere trennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe (R₁=COOR₄) verseift und/oder reduziert und/oder eine Carboxylgruppe (R₄=H) verestert und/oder eine freie Carboxylgruppe (R₄=H) in ein Amid überführt oder eine Carboxygruppe mit einer physiologisch verträglichen Base in ein Salz überführt.

## Claims

1. Leucotriene B₄ derivatives of the formula I 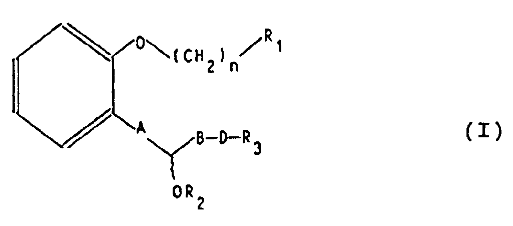 wherein n = 1-10,
R₁ represents the radical CH₂OH, the radical COOR₄ in which R₄ represents a hydrogen atom, an alkyl radical having 1-10 carbon atoms, a cycloalkyl radical having 3-10 carbon atoms, an aryl radical having 6-10 carbon atoms that is optionally mono- or di-substituted by chlorine, bromine, phenyl, alkyl having 1-4 carbon atoms, chloromethyl, fluoromethyl, trifluoromethyl, carboxy, C₁-C₄-alkoxy or by hydroxy, or a CH₂-CO-aryl radical having 6-10 carbon atoms for aryl or a 5- or 6-membered heterocyclic radical having at least 1 hetero atom, or R₁ represents the radical CONHR₅ in which R₅ represents an alkanoyl or alkanesulphonyl radical having 1-10 carbon atoms or the radical R₄, or R₁ represents the radical CONR₆R₇ wherein R₆ and R₇ represent alkyl having 1-10 carbon atoms or together represent an alkylene radical having 3-6 carbon atoms,
A represents a cis,trans- or trans,trans-CH=CH-CH=CH-or tetramethylene group,
B represents a straight-chain or branched saturated or unsaturated alkylene group having up to 10 carbon atoms which may optionally be substituted by fluorine, or represents the group 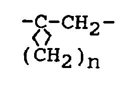 in which n = 1, 2 or 3,
D represents a direct bond, oxygen, sulphur, a -C≡C- group or a -CH=CR₇ group in which R₇ represents hydrogen, C₁-C₅-alkyl, chlorine or bromine,
B and D together represent a direct bond,
R₂ represents a hydrogen atom or an acid residue of an organic acid having 1-15 carbon atoms, and
R₃ represents a hydrogen atom, an alkyl radical having 1-10 carbon atoms, an alkyl radical having 1-10 carbon atoms that is substituted by chlorine or bromine, a cycloalkyl radical having 3-10 carbon atoms, an aryl radical having 6-10 carbon atoms that is optionally mono- or di-substituted by chlorine, bromine, phenyl, alkyl having 1-4 carbon atoms, chloromethyl, fluoromethyl, trifluoromethyl, carboxy, C₁-C₄-alkoxy or by hydroxy, or a 5- or 6-membered heterocyclic radical having at least 1 hetero atom, and, if R₄ represents a hydrogen atom, their salts with physiologically tolerable bases and their cyclodextrin clathrates.

2. 4-[2-[(1E,3E)-(5RS)-5-hydroxy-1,3-tridecadienyl]-phenoxy]-butyric acid ethyl ester.

3. 4-[2-[(1E,3E)-(5R)-5-hydroxy-1,3-tridecadienyl]-phenoxy]-butyric acid ethyl ester.

4. 4-[2-[(1Z,3E)-(5RS)-5-hydroxy-1,3-tridecadienyl]-phenoxy]-butyric acid ethyl ester.

5. 4-[2-[(1E,3E)-(5RS)-5-hydroxy-1,3-tridecadienyl]-phenoxy]-butyric acid methyl ester.

6. 4-[2-[(1E,3E)-(5RS)-5-hydroxy-1,3-tridecadienyl]-phenoxy]-butyric acid isopropyl ester.

7. 4-[2-[(1E,3E)-(5RS)-5-acetoxy-1,3-tridecadienyl]-phenoxy]-butyric acid ethyl ester.

8. 4-[(5RS)-5-hydroxy-tridecyl]-phenoxy]-butyric acid ethyl ester.

9. 4-[2-[(1E,3E)-(5RS)-5-hydroxy-1,3-tridecadienyl]-phenoxy]-butyric acid.

10. 4-[2-[(1Z,3E)-(5RS)-5-hydroxy-1,3-tridecadienyl]-phenoxy]-butyric acid.

11. 4-[2-[(1E,3E)-(5RS)-5-hydroxy-1,3-tridecadienyl]phenoxy]-butan-1-ol.

12. 2-[2-[(1E,3E)-(5RS)-5-hydroxy-1,3-tridecadienyl]-phenoxy]-acetic acid ethyl ester.

13. 2-[2-[(1E,3E)-(5RS)-5-hydroxy-1,3-tridecadienyl]-phenoxy]-acetic acid.

14. 5-[2-[(1E,3E)-(5RS)-5-hydroxy-1,3-tridecadienyl]-phenoxy]-valeric acid ethyl ester.

15. 4-[2-[(1E,3E)-(5RS)-5-hydroxy-1,3-tridecadienyl]-phenoxy]-butyric acid tetramethyleneamide.

16. Process for the manufacture of the leucotriene B₄ derivatives of the formula I according to claim 1, characterised in that a vinyl halide of the formula II or III 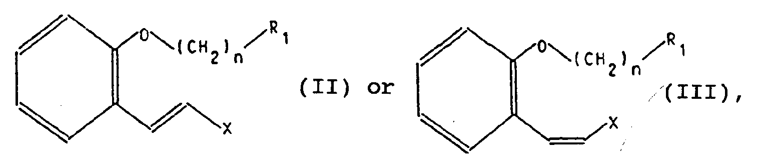 wherein R₁ and n have the meanings already given and X is an iodine or bromine atom, is reacted with an organotin compound of the formula IV 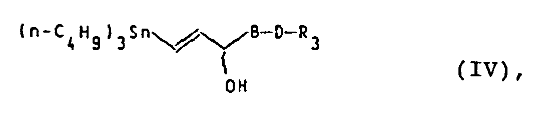 wherein B, D and R₃ have the meanings already given, with the aid of a palladium catalyst, and then optionally, in any desired sequence, enantiomers are separated, protected hydroxy groups are freed and/or a free hydroxy group is esterified and/or the 1-hydroxy group is oxidised to carboxylic acid and/or double bonds are hydrogenated and/or an esterified carboxy group (R₁ = COOR₄) is hydrolysed and/or reduced and/or a carboxy group (R₄ = H) is esterified and/or a free carboxy group (R₄ = H) is converted into an amide, or a carboxy group is converted into a salt with a physiologically tolerable base.

## Revendications

1. Dérivés du leucotriène-B₄ répondant à la formule I : 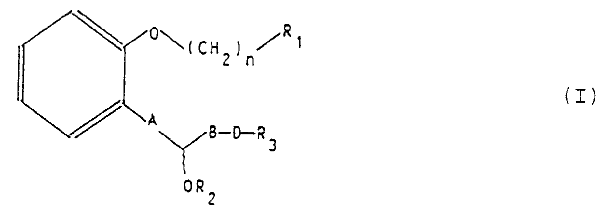 dans laquelle n est un nombre de 1 à 10,
R₁ désigne le radical CH₂OH, le radical COOR₄, dans lequel R₄ désigne un atome d'hydrogène, un radical alkyle en C₁-C₁₀, un radical cycloalkyle en C₃-C₁₀, un radical aryle en C₆-C₁₀ éventuellement substitué par un à deux radicaux chlore, brome, phényle, alkyle en C₁-C₄, chlorométhyle, fluorométhyle, trifluorométhyle, carboxy, alcoxy en C₁-C₄ ou hydroxy, un radical -CH₂-CO-aryle ayant 6 à 10 atomes de carbone pour la partie aryle et un radical hétérocyclique à 5 ou 6 maillons ayant au moins un hétéroatome, le radical CONHR₅ dans lequel R₅ désigne un radical alcanoyle ou alcane-sulfonyle en C₁-C₁₀ ou le radical R₄ ou le radical CONR₆R₇, R₆ et R₇ désignant un groupe alkyle en C₁-C₁₀, ou ensemble un radical alkylène en C₃-C₆,
A désigne un radical cis,trans- ou trans,trans-CH=CH-CH=CH- ou un groupe tétraméthylène,
B désigne un groupe terminal alkylène ayant jusqu'à 10 atomes de carbone, linéaire ou ramifié, saturé ou insaturé, qui peut le cas échéant être substitué par des atomes de fluor, ou le groupe 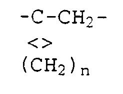 dans lequel n est 1, 2 ou 3,
D désigne une liaison directe, un atome d'oxygène, de soufre, un groupe -C≡C- ou un groupe -CH=CR₇- dans lequel R₇ est un atome d'hydrogène, un groupe alkyle en C₁-C₅, un atome de chlore ou de brome,
B et D désignent ensemble une liaison directe,
R₂ est un atome d'hydrogène ou un radical d'acide d'un acide organique en C₁-C₁₅, et
R₃ est un atome d'hydrogène, un radical alkyle en C₁-C₁₀, un radical alkyle en C₆-C₁₀ substitué par un atome de chlore ou de brome, un radical cycloalkyle en C₃-C₁₀, un radical aryle en C₃-C₁₀ éventuellement substitué par un ou deux radicaux chlore, brome, phényle, alkyle en C₁-C₄, chlorométhyle, fluorométhyle, trifluorométhyle, carboxy, alcoxy en C₁-C₄ ou hydroxy, ou un radical hétérocyclique à 5 ou 6 maillons ayant au moins un hétéroatome, et, si R₄ désigne un atome d'hydrogène, leurs sels avec des bases physiologiquement acceptables et leurs clathrates avec la cyclodextrine.

2. 4-[2-[(1E,3E)-(5RS)-5-Hydroxy-1,3-tridécadiényl]-phénoxy]-butyrate d'éthyle

3. 4-[2-[(1E,3E)-(5R)-5-Hydroxy-1,3-tridécadiényl]-phénoxy]-butyrate d'éthyle

4. 4-[2-[(1Z,3E)-(5RS)-5-Hydroxy-1,3-tridécadiényl]-phénoxy]-butyrate d'éthyle

5. 4-[2-[(1E,3E)-(5RS)-5-Hydroxy-1,3-tridécadiényl]-phénoxy]-butyrate de méthyle

6. 4-[2-[(1E,3E)-(5RS)-5-Hydroxy-1,3-tridécadiényl]-phénoxy]-butyrate d'isopropyle

7. 4-[2-[(1E,3E)-(5RS)-5-Acétoxy-1,3-tridécadiényl]-phénoxy]-butyrate d'éthyle

8. 4-[(5RS)-5-Hydroxy-tridécyl]-phénoxy]-butyrate d'éthyle

9. Acide 4-[2-[(1E,3E)-(5RS)-5-hydroxy-1,3-tridécadiényl]-phénoxy]-butyrique

10. Acide 4-[2-[(1Z,3E)-(5RS)-5-hydroxy-1,3-tridécadiényl]-phénoxy]-butyrique

11. 4-[2-[(1E,3E)-(5RS)-5-Hydroxy-1,3-tridécadiényl]-phénoxy]-butan-1-ol

12. 2-[2-[(1E,3E)-(5RS)-5-Hydroxy-1,3-tridécadiényl]-phénoxy]-acétate d'éthyle

13. Acide 2-[2-[(1E,3E)-(5RS)-5-hydroxy-1,3-tridécadiényl]-phényl]-acétique

14. 5-(2-[(1E,3E)-(5RS)-5-Hydroxy-1,3-tridécadiényl]-phénoxy]-valérate d'éthyle

15. Tétraméthylènamide de l'acide 4-[2-[(1E,3E)-(5RS)-5-hydroxy-1,3-tridécadiényl]-phénoxy)-butyrique

16. Procédé de préparation des dérivés du leucotriène-B₄ répondant à la formule I selon la revendication 1, caractérisé en ce que l'on fait réagir un halogénure de vinyle répondant aux formules II ou III : 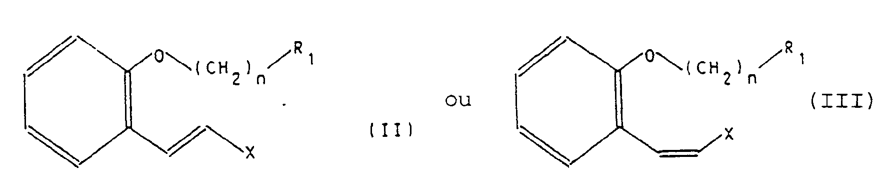 dans lesquelles R₁ et n ont les significations déjà indiquées et X est un atome d'iode ou de brome, avec un composé organique de l'étain répondant à la formule IV : 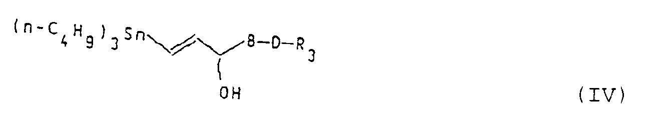 dans laquelle B, D et R₃ ont les significations déjà indiquées, à l'aide d'un catalyseur à base de palladium, puis le cas échéant, dans un ordre quelconque, on sépare les énantiomères, on libère les groupes hydroxy protégés, et/ou on estérifie un groupe hydroxy libre et/ou on oxyde le groupe 1-hydroxy en acide carboxylique et/ou on hydrogène des doubles liaisons et/ou on saponifie et/ou on réduit un groupe carboxyle estérifié (R₁ = COOR₄) et/ou on estérifie un groupe carboxyle (R₄ = H) et/ou on transforme un groupe carboxyle libre (R₄ = H) en un amide ou on transforme un groupe carboxy en sel avec une base physiologiquement acceptable.
